# EUROPÄISCHE PATENTSCHRIFT

(11) **EP 1 124 809 B1**
(45) Veröffentlichungstag und Bekanntmachung des Hinweises auf die Patenterteilung: **24.03.2004**
(21) Anmeldenummer: 99950775.9
(22) Anmeldetag: 23.10.1999
(51) Int. Cl.: C07D 273/04, C07D 401/12, A61K 31/50, A61K 31/501, A61P 19/10, A61P 11/06

(54) **BENZOYLPYRIDAZINE**
BENZOYLPYRIDAZINES
BENZOYLPYRIDAZINES

(30) Priorität: 04.11.1998 DE 19850701
(43) Veröffentlichungstag der Anmeldung: 22.08.2001
(73) Patentinhaber: MERCK PATENT GmbH, 64293 Darmstadt (DE)
(72) Erfinder: JONAS, Rochus, D-64291 Darmstadt (DE); WOLF, Michael, D-64297 Darmstadt (DE); KLUXEN, Franz-Werner, D-64285 Darmstadt (DE)
(74) Vertreter: Isenbruck, Günter, Dr.
(86) Internationale Anmeldenummer: PCT/EP1999/008047
(87) Internationale Veröffentlichungsnummer: WO 2000/026201

(56) Entgegenhaltungen:
- EP-A- 0 738 715
- WO-A-94/01412
- WO-A-98/06704
- COMBS D W ET AL: "NONSTEROIDAL PROGESTERONE RECEPTOR LIGANDS. 1. 3-ARYL-1-BENZOYL-1,4,5,6-TETRAHYDROPYRIDAZ INES" JOURNAL OF MEDICINAL CHEMISTRY,US,AMERICAN CHEMICAL SOCIETY. WASHINGTON, Bd. 38, Nr. 25, Seite 4878-4879 XP002047380 ISSN: 0022-2623

## Beschreibung

Die Erfindung betrifft Benzoylpyridazinderivate der Formel I 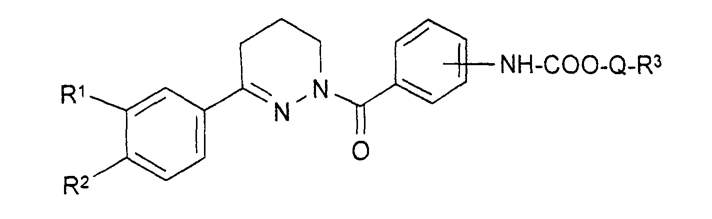 worin
- R¹, R²: jeweils unabhängig voneinander -OH, OR⁵, -S-R⁵, -SO-R⁵, -SO₂-R⁵ oder Hal,
- R¹ und R²: zusammen auch -O-CH₂-O-,
- R³: NH₂, NHA, NAA' oder einen gesättigten Heterocyclus mit 1 bis 4 N-, O- und/oder S-Atomen, der unsubstituiert oder ein-, zwei- oder dreifach durch Hal, A und/oder OA substituiert sein kann,
- Q: fehlt oder verzweigtes oder unverzweigtes Alkylen mit 1-10 C-Atomen,
- R⁵: A, Cycloalkyl mit 3-7 C-Atomen, Alkylencycloalkyl mit 4-8 C-Atomen oder Alkenyl mit 2-8 C-Atomen,
- A, A': jeweils unabhängig voneinander Alkyl mit 1 bis 10 C-Atomen, das durch 1 bis 5 F- und/oder Cl-Atome substituiert sein kann und
- Hal: F, Cl, Br oder I
bedeuten,
sowie deren physiologisch unbedenklichen Salze und Solvate.

1-Benzoyl-tetrahydropyridazine als Progesteron-Rezeptorliganden sind z.B. in J. Med.Chem. 38, 4878 (1995) beschrieben.
Andere Arylalkanoylpyridazine sind z.B. aus der DE 196 32 549 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen.

Insbesondere zeigen sie eine selektive Phosphodiesterase IV-Hemmung, die mit einer intrazellulären Erhöhung von cAMP verbunden ist (N. Sommer et al., Nature Medicine, 1, 244-248 (1995)).
Die PDE IV-Hemmung kann z.B. analog C.W. Davis in Biochim. biophys. Acta 797, 354-362 (1984) nachgewiesen werden.

Die erfindungsgemäßen Verbindungen können zur Behandlung von asthmatischen Erkrankungen eingesetzt werden. Die antiasthmatische Wirkung der PDE IV-Hemmer ist z.B. von T.J. Torphy et al. in Thorax, **46,** 512-523 (1991) beschrieben und kann z. B. nach der Methode von T. Olsson, Acta allergologica **26,** 438-447 (1971), bestimmt werden.

Da cAMP knochenabbauende Zellen hemmt und knochenaufbauende Zellen stimuliert (S. Kasugai et al., M681 und K. Miyamoto, M 682, in Abstract der American Society for Bone and Mineral Research 18^{th} annual meeting, 1996), können die erfindungsgemäßen Verbindungen zur Behandlung von Osteoporose eingesetzt werden.

Die Verbindungen zeigen außerdem eine hemmende Wirkung auf die Bildung von TNF (Tumor Nekrose Faktor) und eignen sich daher zur Behandlung von allergischen und entzündlichen Krankheiten, Autoimmunkrankheiten und Transplantatabstoßungsreaktionen.

Sie können zur Behandlung von Gedächtnisstörungen, Tumoren, Kachexie, Atherosklerose, rheumatoide Arthritis, multiple Sklerose, Morbus Crohn, atopische Dermatitis, Diabetes mellitus, Ulzerative Kolitis und AIDS eingesetzt werden.

Die Wirkung von PDE IV-Hemmern bei der Behandlung von Asthma, entzündlichen Erkrankungen, Diabetes mellitus, atopischer Dermatitis, Psoriasis, AIDS, Tumorwachstum oder Tumormetastasen ist z.B. in der EP 77 92 91 beschrieben.

Die antiinflammatorische Wirkung der erfindungsgemäßen Substanzen und ihre Wirksamkeit zur Behandlung von z.B. Autoimmunerkrankungen, Multiplesklerose oder rheumatoider Arthritis kann analog den Methoden von N. Sommer et al., Nature Medicine, **1**, 244-248 (1995) oder L. Sekut et al., Clin. Exp. Immunol., **100**, 126-132 (1995) bestimmt werden.

Die Wirkung von PDE IV-Hemmern bei der Tumorbehandlung ist z.B. in der WO 95 35 281, WO 95 17 399 oder WO 96 00 215 beschrieben.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Gegenstand der Erfindung sind dementsprechend die Verbindungen der Formel I sowie ein Verfahren zur Herstellung von Verbindungen der Formel I nach Anspruch 1 sowie deren Salzen, dadurch gekennzeichnet, daß man eine Verbindung der Formel II 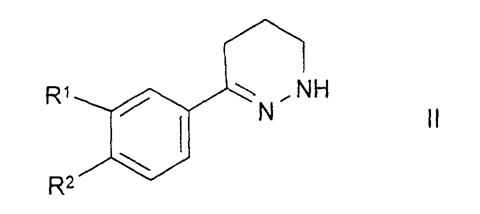 worin
R¹ und R² die angegebenen in Anspruch 1 angegebenen Bedeutungen haben,
mit einer Verbindung der Formel III 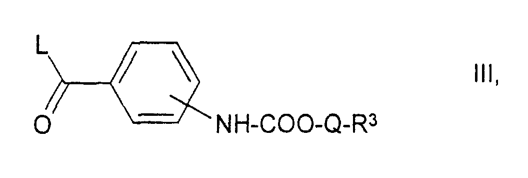 worin
Q und R³ die in Anspruch 1 angegebenen Bedeutungen haben, und
L Cl, Br, OH oder eine reaktionsfähige veresterte OH-Gruppe bedeutet,
umsetzt,
oder
daß man eine Verbindung der Formel IV 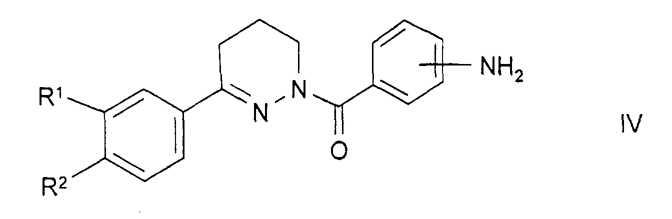 worin
R¹ und R² die angegebenen Bedeutungen haben, mit einer Verbindung der Formel V

R³-Q-O-CO-L V

worin
R³ und Q die angegebenen Bedeutungen haben, und
L Cl, Br, OH oder eine reaktionsfähige veresterte OH-Gruppe
bedeutet,
umsetzt,
und/oder daß man eine basische Verbindung der Formel I durch Behandeln mit einer Säure in eines ihrer Salze überführt.

Vor- und nachstehend haben die Reste R¹, R², R³, Q und L die bei den Formeln I, II, III, IV und V angegebenen Bedeutungen, sofern nicht ausdrücklich etwas anderes angegeben ist.

Die Verbindungen der Formel I können ein chirales Zentrum aufweisen und können daher in mehreren stereoisomeren Formen auftreten. Alle diese Formen (z. B. R- und S-Formen) und deren Gemische (z. B. die R,S-Formen) sind in der Formel I eingeschlossen.
Unter Solvaten versteht man z.B. die Hydrate oder Alkoholate der Verbindungen der Formel I.

A und A' bedeutet vorzugsweise Alkyl, weiter bevorzugt durch 1 bis 5 Fluor- und/oder Chloratome substituiertes Alkyl.

In den vorstehenden Formeln ist Alkyl vorzugsweise unverzweigt und hat 1, 2, 3, 4, 5, 6, 7, 8, 9 oder 10 C-Atome, vorzugsweise 1, 2, 3, 4 oder 5 C-Atome und bedeutet vorzugsweise Methyl, Ethyl, Trifluormethyl, Pentafluorethyl oder Propyl, weiterhin bevorzugt Isopropyl, Butyl, Isobutyl, sek.-Butyl oder tert.-Butyl, aber auch n-Pentyl, neo-Pentyl oder Isopentyl.

Cycloalkyl hat vorzugsweise 3-7 C-Atome und steht bevorzugt für Cyclopropyl und Cyclobutyl, weiterhin bevorzugt für Cyclopentyl oder Cyclohexyl, ferner auch für Cycloheptyl.

Alkylencycloalkyl hat vorzugsweise 4-8 C-Atome und steht bevorzugt für Methylencyclopropyl und Methylencyclobutyl, weiterhin bevorzugt für Methylencyclopentyl und Methylencyclohexyl, ferner auch für Methylencycloheptyl.

Alkenyl steht vorzugsweise für Vinyl, 1- oder 2-Propenyl, 1-Butenyl, Isobutenyl, sek.-Butenyl, ferner bevorzugt ist 1-Pentenyl, iso-Pentenyl oder 1-Hexenyl.

Alkylen ist unverzweigt oder verzweigt und bedeutet bevorzugt Methylen, Ethylen, ferner bevorzugt Propylen, Butylen, Pentylen, Hexylen, ferner Heptylen, Octylen, Nonylen oder Decylen.

Hal bedeutet vorzugsweise F, Cl oder Br, aber auch I.

Die Reste R¹ und R² können gleich oder verschieden sein und stehen in der 3- oder 4-Position des Phenylrings. Sie bedeuten beispielsweise unabhängig voneinander Hydroxy, -S-CH₃, -SO-CH₃, -SO₂CH₃, F, Cl, Br oder I oder zusammen Methylendioxy. Besonders bevorzugt stehen sie aber jeweils für Methoxy, Ethoxy, Propoxy, Isopropoxy, Cyclopentoxy, oder aber für Fluor-, Difluor-, Trifluormethoxy, 1-Fluor-, 2-Fluor-, 1,2-Difluor-, 2,2-Difluor-, 1,2,2-Trifluor- oder 2,2,2-Trifluorethoxy.
R¹ bedeutet ganz besonders bevorzugt Methyl, Ethyl oder Isopropyl. R² bedeutet ganz besonders bevorzugt Methyl.

Q bedeutet vorzugsweise z.B. eine Bindung, Methylen, Ethylen, Propylen, Butylen oder Pentylen.

Heterocyclus bedeutet vorzugsweise z.B. Piperidinyl, Tetrahydrofuranyl oder Pyrrolidinyl.

Der Rest R³ bedeutet vorzugsweise weiterhin Amino, N-Methylamino, N,N-Dimethylamino, N-Ethylamino, N,N-Diethylamino oder N-Methyl-piperidin-4-yl.

Für die gesamte Erfindung gilt, daß sämtliche Reste, die mehrfach auftreten, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis le ausgedrückt werden, die der Formel I entsprechen und worin die nicht näher bezeichneten Reste die bei der Formel I angegebene Bedeutung haben, worin jedoch
in la
   - R¹ und R²: jeweils unabhängig voneinander OA,
   - Q: Alkylen mit 1-6 C-Atomen und
   - R³: unsubstituiertes oder ein- oder zweifach durch Hal oder A substituiertes Piperidinyl, Pyrazinyl oder Pyrrolidinyl,
   bedeuten;
in Ib
   - R¹ und R²: jeweils unabhängig voneinander OA,
   - Q: fehlt oder Alkylen mit 1-6 C-Atomen und
   - R³: unsubstituiertes oder ein- oder zweifach durch Hal oder A substituiertes Piperidinyl oder Pyrrolidinyl, NH₂, NHA oder NAA',
   bedeuten;
in Ic
   - R¹ und R²: zusammen -O-CH₂-O-,
   - Q: fehlt oder Alkylen mit 1-6 C-Atomen und
   - R³: unsubstituiertes oder ein- oder zweifach durch Hal oder A substituiertes Piperidinyl oder Pyrrolidinyl, NH₂, NHA oder NAA',
   bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II und IV haben R¹ und R² die angegebenen Bedeutungen, insbesondere die angegebenen bevorzugten Bedeutungen.

In den Verbindungen der Formeln III und V steht Q vorzugsweise für Methylen oder Ethylen, ferner bevorzugt für Propylen oder Butylen.

R³ hat in den Verbindungen der Formeln III und V die angegebenen bevorzugten Bedeutungen, während L Cl, Br, OH oder eine reaktionsfähige veresterte OH-Gruppe bedeutet.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyloder p-Tolylsulfonyloxy, ferner auch 2-Naphthalinsulfonyloxy).

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
Andererseits ist es möglich, die Reaktion stufenweise durchzuführen.

Die Verbindungen der Formel I können vorzugsweise erhalten werden, indem man Verbindungen der Formel II mit Verbindungen der Formel III umsetzt.

Die Ausgangsstoffe der Formeln II und III sind teilweise bekannt. Sofern sie nicht bekannt sind, können sie nach an sich bekannten Methoden hergestellt werden.

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Als inerte Lösungsmittel eignen sich z.B. Kohlenwasserstoffe wie Hexan, Petrolether, Benzol, Toluol oder Xylol; chlorierte Kohlenwassertoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.

Verbindungen der Formel I können weiterhin erhalten werden, indem man Verbindungen der Formel IV mit Verbindungen der Formel V umsetzt.
Die Ausgangsverbindungen der Formeln IV und V sind in der Regel bekannt. Sind sie nicht bekannt, so können sie nach an sich bekannten Methoden hergestellt werden.
So ist z.B. die Herstellung von 1-Benzoyl-tetrahydropyridazin in J. Med. Chem. 38, 4878 (1995) beschrieben.

In den Verbindungen der Formel V bedeutet der Rest -CO-L eine voraktivierte Carbonsäure, vorzugsweise ein Carbonsäurehalogenid.

Die Umsetzung der Verbindungen der Formel IV mit Verbindungen der Formel Verfolgt unter den gleichen Bedingungen, betreffend die Reaktionszeit, Temperatur und Lösungsmittel, wie dies für die Umsetzung der Verbindungen der Formel II mit Verbindungen der Formel III beschrieben ist.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Andererseits können, falls gewünscht, die freien Basen der Formel I aus ihren Salzen mit Basen (z.B. Natrium- oder Kaliumhydroxid oder - carbonat) in Freiheit gesetzt werden.

Gegenstand der Erfindung ist ferner die Verwendung der Verbindungen der Formel I und/oder ihrer physiologisch unbedenklichen Salze zur Herstellung pharmazeutischer Zubereitungen, insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind auch Arzneimittel der Formel I und ihre physiologisch unbedenklichen Salze als Phosphodiesterase IV-Hemmer.

Gegenstand der Erfindung sind ferner pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder ein oder mehrere weitere Wirkstoffe enthalten, z.B. ein oder mehrere Vitamine.

Die Verbindungen der Formel I und ihre physiologisch unbedenklichen Salze können bei der Bekämpfung von Krankheiten, bei denen eine Erhöhung des cAMP(cyclo-Adenosin-monophosphat)-Spiegels zu Entzündungshemmung oder -verhinderung und Muskelentspannung führt, eingesetzt werden. Besondere Verwendung können die erfindungsgemäßen Verbindungen bei der Behandlung von Allergien, Asthma, chronischer Bronchitis, atopischer Dermatitis, Psoriasis und anderer Hautkrankheiten und Autoimmunerkrankungen finden.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre Enantiomeren getrennt werden. Vorzugsweise werden aus dem racemischen Gemisch durch Umsetzung mit einem optisch-aktiven Trennmittel Diastereomere gebildet.

Natürlich ist es auch möglich, optisch aktive Verbindungen der Formel I nach den oben beschriebenen Methoden zu erhalten, indem man Ausgangsstoffe verwendet, die bereits optisch aktiv sind.

Die Formel I umschließt alle Stereoisomeren und deren Gemische, z.B. die Racemate.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.
- Massenspektrometrie (MS):: El (Elektronenstoß-Ionisation) M⁺
FAB (Fast Atom Bombardment) (M+H)⁺

### Beispiel 1

Eine Lösung von 1,40 g 1-(4-Aminobenzoyl)-3-(3-ethoxy-4-methoxyphenyl)-1,4,5,6-tetrahydropyridazin ("AB") in 80 ml Dichlormethan und 0,8 ml Pyridin wird mit 0,9 g Chlorameisensäuredimethylaminopropylester in 15 ml Dichlormethan versetzt und 2 Stunden nachgerührt. Man arbeitet wie üblich auf und erhält 1,6 g {4-[3-(3-Ethoxy-4-methoxyphenyl)-1,4,5,6-tetrahydropyridazin-1-ylcarbonyl]-phenyl}-carbaminsäure-(3-dimethylaminopropyl)-ester, Hemifumarat, F. 193°.

Analog erhält man durch Umsetzung von "AB"
mit Chlorameisensäure-(N-methylpiperidin-4-yl)-ester:
{4-[3-(3-Ethoxy-4-methoxyphenyl)-1,4,5,6-tetrahydropyridazin-1-ylcarbonyl]-phenyl}-carbaminsäure-(N-methylpiperidin-4-yl)-ester, Hydrochlorid, F. 243°.

Analog erhält man durch Umsetzung
von 1-(4-Aminobenzoyl)-3-(3-Isopropoxy-4-methoxyphenyl)-1,4,5,6-tetrahydropyridazin
mit Chlorameisensäuredimethylaminopropylester
{4-[3-(3-Isopropoxy-4-methoxyphenyl)-1,4,5,6-tetrahydropyridazin-1-ylcarbonyl]-phenyl}-carbaminsäure-(3-dimethylaminopropyl)-ester; Hydrochlorid, F. 213°.

Analog erhält man durch Umsetzung
von 1-(3-Aminobenzoyl)-3-(3-ethoxy-4-methoxyphenyl)-1,4,5,6-tetrahydropyridazin
mit Chlorameisensäuredimethylaminopropylester
{3-[3-{3-Ethoxy-4-methoxyphenyl)-1,4,5,6-tetrahydropyridazin-1-ylcarbonyl]-phenyl}-carbaminsäure-(3-dimethylaminopropyl)-ester, Fumarat, F. 181°.

### Beispiel 2

Analog Beispiel 1 erhält man die Verbindungen
{3-[3-(3-Cyclopentyloxy-4-methoxyphenyl)-1,4,5,6-tetrahydropyridazin-1-ylcarbonyl]-phenyl}-darbaminsäure-(3-dimethylaminopropyl)-ester, Hydrochlorid, F. 235°;
{3-[3-(3-Cyclopentyloxy-4-methoxyphenyl)-1,4,5,6-tetrahydropyridazin-1-ylcarbonyl]-phenyl}-carbaminsäure-(N-methylpiperidin-4-yl)-ester,
{3-[3-(3-Propyloxy-4-methoxyphenyl)-1,4,5,6-tetrahydropyridazin-1-ylcarbonyl]-phenyl}-carbaminsäure-(3-dimethylaminopropyl)-ester, Hydrochlorid, F. 189°;
{4-[3-(3,4-Diethoxyphenyl)-1,4,5,6-tetrahydropyridazin-1-ylcarbonyl]-phenyl}-carbaminsäure-(3-dimethylaminopropyl)-ester, Hydrochlorid, F. 175°;
{4-[3-(3,4-Diethoxyphenyl)-1,4,5,6-tetrahydropyridazin-1-ylcarbonyl]-phenyl}-carbaminsäure-(N-methylpiperidin-4-yl)-ester, Fumarat, F. 202°;
{3-[3-(3,4-Dimethoxyphenyl)-1,4,5,6-tetrahydropyridazin-1-ylcarbonyl]-phenyl}-carbaminsäure-(3-dimethylaminopropyl)-ester, Hydrochlorid, F. 204°;
{4-[3-(3,4-Dimethoxyphenyl)-1,4,5,6-tetrahydropyridazin-1-ylcarbonyl]-phenyl}-carbaminsäure-(3-dimethylaminopropyl)-ester, Fumarat, F. 180°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 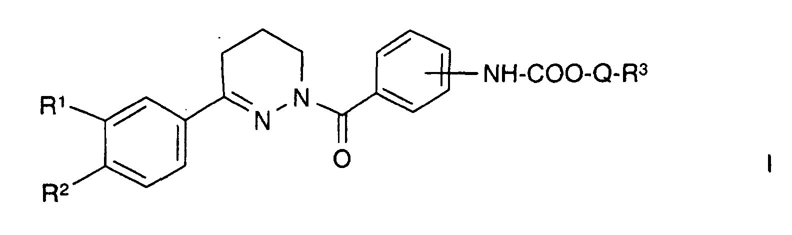 worin
R¹, R² jeweils unabhängig voneinander -OH, OR⁵, -S-R⁵, -SO-R⁵, -SO₂-R⁵ oder Hal,
R¹ und R² zusammen auch -O-CH₂-O-,
R³ NH₂, NHA, NAA' oder einen gesättigten Heterocyclus mit 1 bis 4 N-, O- und/oder S-Atomen, der unsubstituiert oder ein-, zwei- oder dreifach durch Hal, A und/oder OA substituiert sein kann,
Q fehlt oder verzweigtes oder unverzweigtes Alkylen mit 1-10 C-Atomen,
R⁵ A, Cycloalkyl mit 3-7 C-Atomen, Alkylencycloalkyl mit 4-8 C-Atomen oder Alkenyl mit 2-8 C-Atomen,
A, A' jeweils unabhängig voneinander Alkyl mit 1 bis 10 C-Atomen, das durch 1 bis 5 F- und/oder Cl-Atome substituiert sein kann und
Hal F, Cl, Br oder I
bedeuten,
sowie deren physiologisch unbedenklichen Salze und Solvate.

2. Verbindungen der Formel I gemäß Anspruch 1
(a) {4-[3-(3-Ethoxy-4-methoxyphenyl)-1,4,5,6-tetrahydropyridazin-1-ylcarbonyl]-phenyl}-carbaminsäure-(3-dimethylaminopropyl)-ester;
(b) {4-[3-(3-Ethoxy-4-methoxyphenyl)-1,4,5,6-tetrahydropyridazin-1-ylcarbonyl]-phenyl}-carbaminsäure-(N-methylpiperidin-4-yl)-ester;
(c) {4-[3-(3-Isopropoxy-4-methoxyphenyl)-1,4,5,6-tetrahydropyridazin-1-ylcarbonyl]-phenyl}-carbaminsäure-(3-dimethylaminopropyl)-ester;
(d) {3-[3-(3-Ethoxy-4-methoxyphenyl)-1,4,5,6-tetrahydropyridazin-1-ylcarbonyl]-phenyl}-carbaminsäure-(3-dimethylaminopropyl)-ester;
sowie deren physiologisch unbedenklichen Salze und Solvate.

3. Verfahren zur Herstellung von Verbindungen der Formel I nach Anspruch 1 sowie deren Salzen, **dadurch gekennzeichnet, daß** man eine Verbindung der Formel II 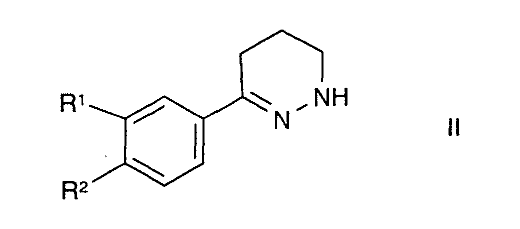 worin
R¹ und R² die angegebenen in Anspruch 1 angegebenen Bedeutungen haben,
mit einer Verbindung der Formel III 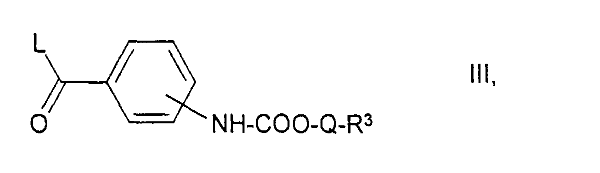 worin
Q und R³ die in Anspruch 1 angegebenen Bedeutungen haben,
und
L Cl, Br, OH oder eine reaktionsfähige veresterte OH-Gruppe bedeutet,
umsetzt,
oder
daß man eine Verbindung der Formel IV 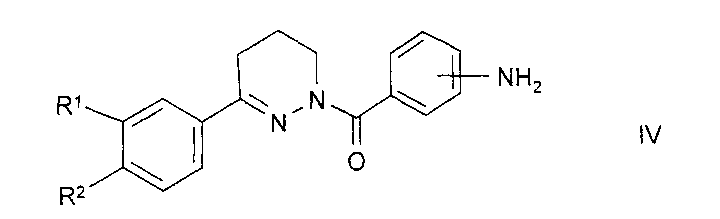 worin
R¹ und R² die angegebenen Bedeutungen haben, mit einer Verbindung der Formel V
R³-Q-O-CO-L v
worin
R³ und Q die angegebenen Bedeutungen haben, und
L Cl, Br, OH oder eine reaktionsfähige veresterte OH-Gruppe bedeutet,
umsetzt,
und/oder daß man eine basische Verbindung der Formel I durch Behandeln mit einer Säure in eines ihrer Salze überführt.

4. Verfahren zur Herstellung pharmazeutischer Zubereitungen, **dadurch gekennzeichnet, daß** man eine Verbindung der Formel I nach Anspruch 1 und/oder eines ihrer physiologischen unbedenklichen Salze zusammen mit mindestens einem festen, flüssigen oder halbflüssigen Träger- oder Hilfsstoff in eine geeignete Dosierungsform bringt.

5. Pharmazeutische Zubereitung, **gekennzeichnet durch** einen Gehalt an mindestens einer Verbindung der Formel I nach Anspruch 1 und/oder einem ihrer physiologisch unbedenklichen Salze.

6. Verbindungen der Formel I nach Anspruch 1 und ihre physiologisch unbedenklichen Salze zur Bekämpfung von Osteoporose, Tumoren, Kachexie, Atherosklerose, rheumatoide Arthritis, multiple Sklerose, Diabetes mellitus, ulzerative Kolitis, entzündlichen Krankheiten, Allergien, Asthma, Autoimmunerkrankungen, AIDS und Transplantatabstoßungsreaktionen.

7. Arzneimittel der Formel I nach Anspruch 1 und ihre physiologisch unbedenklichen Salze als Phosphodiesterase IV-Hemmer.

8. Verwendung von Verbindungen der Formel I nach Anspruch 1 und/oder ihre physiologisch unbedenklichen Salze zur Herstellung eines Arzneimittels.

9. Verbindungen der Formel I nach Anspruch 1 und/oder ihrer physiologisch unbedenklichen Salze zur Verwendung bei der Bekämpfung von Krankheiten.

## Claims

1. Compounds of the formula I 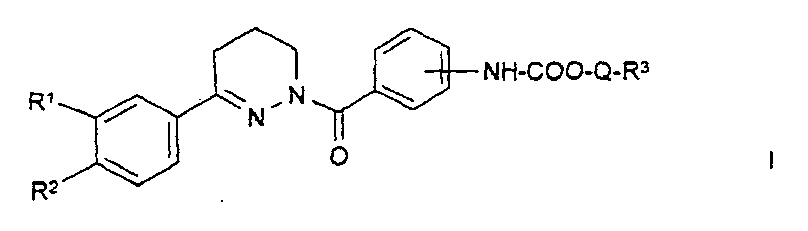 in which
R¹, R² in each case independently of one another are -OH, OR⁵, -S-R⁵, -SO-R⁵, -SO₂-R⁵ or Hal,
R¹ and R² together are also -O-CH₂-O-,
R³ is NH₂, NHa, NAA' or a saturated heterocycle having 1 to 4 N, O and/or S atoms which can be unsubstituted or mono-, di- or trisubstituted by Hal, A and/or OA,
Q is absent or is branched or unbranched alkylene having 1-10 C atoms,
R⁵ is A, cycloalkyl having 3-7 C atoms, alkylenecycloalkyl having 4-8 C atoms or alkenyl having 2-8 C atoms,
A, A' in each case independently of one another are alkyl which has 1 to 10 C atoms and which can be substituted by 1 to 5 F and/or Cl atoms and
Hal is F, Cl, Br or I,
and the physiologically acceptable salts and solvates thereof.

2. Compounds of the formula I according to Claim 1
(a) 3-dimethylaminopropyl {4-[3-(3-ethoxy-4-methoxyphenyl)-1,4,5,6-tetrahydropyridazin-1-ylcarbonyl]-phenyl}carbamate;
(b) N-methylpiperidin-4-yl-{4-[3-(3-ethoxy-4-methoxyphenyl)-1,4,5,6-tetrahydropyridazin-1-ylcarbonyl]-phenyl}carbamate;
(c) 3-dimethylaminopropyl {4-[3-(3-isopropoxy-4-methoxyphenyl)-1,4,5,6-tetrahydropyridazin-1-ylcarbonyl]phenyl}carbamate;
(d) 3-dimethylaminopropyl {3-[3-(3-ethoxy-4-methoxyphenyl)-1,4,5,6-tetrahydropyridazin-1-ylcarbonyl]-phenyl}carbamate;
and the physiologically acceptable salts and solvates thereof.

3. Process for the preparation of compounds of the formula I according to Claim 1 and salts thereof, **characterized in that** a compound of the formula II 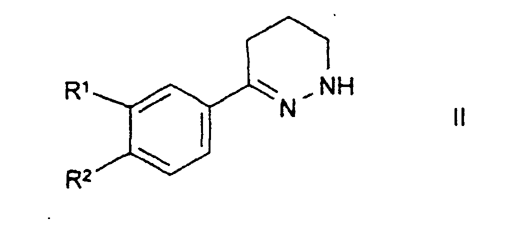 in which
R¹ and R² have the meanings given in Claim 1
is reacted with a compound of the formula III 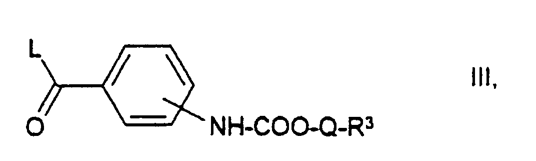 in which
Q and R³ have the meanings given in Claim 1 and
L is Cl, Br, OH or a reactive esterified OH group,
or
**in that** a compound of the formula IV 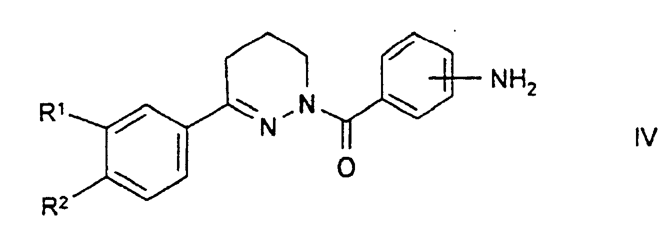 in which
R¹ and R² have the abovementioned meanings
is reacted with a compound of the formula V
R³-Q-O-CO-L V
in which
R³ and Q have the abovementioned meanings and
L is Cl, Br, OH or a reactive esterified OH group,
and/or **in that** a basic compound of the formula I is converted into a salt thereof by treatment with an acid.

4. Process for the preparation of pharmaceutical products, **characterized in that** a compound of the formula I according to Claim 1 and/or a physiologically acceptable salt thereof is brought into a suitable pharmaceutical form together with at least one solid, liquid or semi-liquid excipient or auxiliary.

5. Pharmaceutical product, **characterized in that** it comprises at least one compound of the formula I according to Claim 1 and/or a physiologically acceptable salt thereof.

6. Compounds of the formula I according to Claim 1 and the physiologically acceptable salts thereof for combating osteoporosis, tumours, cachexia, atherosclerosis, rheumatoid arthritis, multiple sclerosis, diabetes mellitus, ulcerative colitis, inflammatory diseases, allergies, asthma, autoimmune diseases, AIDS and transplant rejection reactions.

7. Pharmaceuticals of the formula I according to Claim 1 and the physiologically acceptable salts thereof as phosphodiesterase IV inhibitors.

8. Use of compounds of the formula I according to Claim 1 and/or of the physiologically acceptable salts thereof for the preparation of a pharmaceutical.

9. Compounds of the formula I according to Claim 1 and/or of the physiologically acceptable salts thereof for use for combating diseases.

## Revendications

1. Composés de formule I 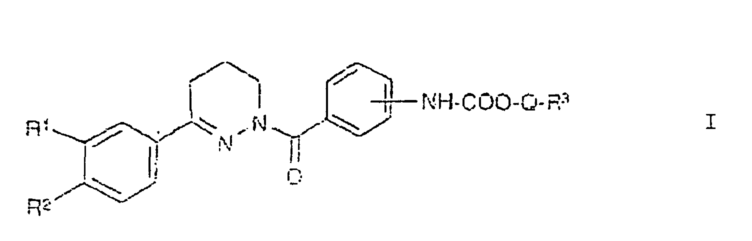 dans laquelle
R¹, R² représentent chacun indépendamment l'un de l'autre -OH, -OR⁵, -S-R⁵, -SO-R⁵, -SO₂-R⁵ ou Hal,
R¹ et R² représentent ensemble aussi -O-CH₂-O-,
R³ représente NH₂, NHA, NAA' ou un hétérocycle saturé avec 1 à 4 atomes de N, de O et/ou de S, qui peut être non substitué ou substitué une, deux ou trois fois par Hal, A et/ou OA,
Q manque ou représente un alkylène ramifié ou non ramifié avec 1 à 10 atomes de C,
R⁵ représente A, un cycloalkyle avec 3 à 7 atomes de C, un cycloalkyle d'alkylène avec 4 à 8 atomes de C ou un alcényle avec 2 à 8 atomes de C,
A, A' représentent chacun indépendamment l'un de l'autre un alkyle avec 1 à 10 atomes de C, qui peut être substitué par 1 à 5 atomes de F et/ou de Cl et Hal représente F, Cl, Br ou I
ainsi que les sels et solvates physiologiquement inoffensifs de ceux-ci.

2. Composés de formule I selon la revendication 1
(a) (3-diméthylaminopropyl)-ester d'acide {4-[3-(3-éthoxy-4-méthoxyphényl)-1,4,5,6-tétrahydropyridazin-1-ylcarbonyl]-phényl}-carbamique ;
(b) (N-méthylpipéridin-4-yl)-ester d'acide {4-[3-(3-éthoxy-4-méthoxyphényl)-1,4,5,6-tétrahydropyridazin-1-ylcarbonyl]-phényl}-carbamique ;
(c) (3-diméthylaminopropyl)ester d'acide {4-[3-(3-isopropoxy-4-méthoxyphényl)-1,4,5,6-tétrahydropyridazin-1-ylcarbonyl]-phényl}-carbamique ;
(d) (3-diméthylaminopropyl)ester d'acide {3-[3-(3-éthoxy-4-méthoxyphényl)-1,4,5,6-tétrahydropyridazin-1-ylcarbonyl]-phényl}-carbamique ;
ainsi que les sels et solvates physiologiquement inoffensifs de ceux-ci.

3. Procédé de fabrication de composés de formule I selon la revendication 1 ainsi que les sels de ceux-ci, **caractérisé en ce que** l'on fait réagir un composé de formule II 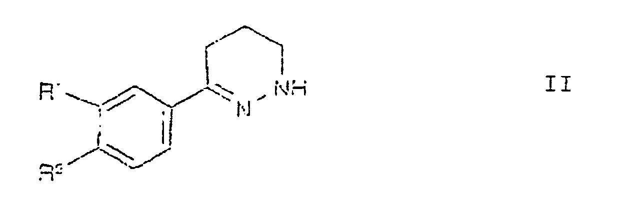 dans laquelle
R¹, R² ont les significations données dans la revendication 1,
avec un composé de formule III 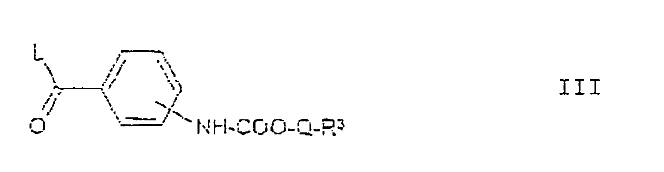 dans laquelle
Q et R³ ont les significations données dans la revendication 1, et
L représente Cl, Br, OH ou un groupe OH estérifié réactif,
ou
**en ce que** l'on fait réagir un composé de formule IV 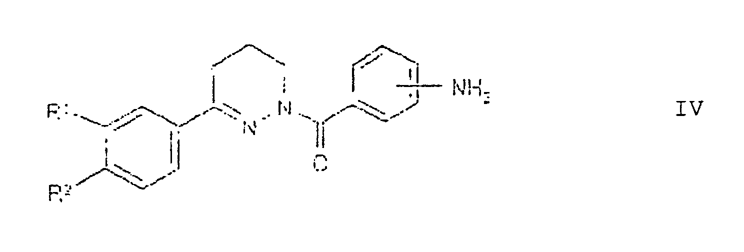 dans laquelle
R¹ et R² ont les significations données, avec un composé de formule V
R³-Q-O-CO-L V
dans laquelle
R³ et Q ont les significations données, et
L représente Cl, Br, OH ou un groupe OH estérifié réactif,
ou/et **en ce que** l'on transforme un composé basique de formule I par traitement avec un acide en un des sels de celui-ci.

4. Procédé de fabrication de préparations pharmaceutiques, **caractérisé en ce que** l'on amène un composé de formule I selon la revendication 1 et/ou un des sels physiologiquement inoffensifs de celui-ci conjointement avec au moins un agent porteur ou correcteur, solide, liquide ou semi-liquide sous une forme de dosage appropriée.

5. Préparation pharmaceutique **caractérisée par** une teneur en au moins un composé de formule I selon la revendication 1 et/ou un des sels physiologiquement inoffensifs de celui-ci.

6. Composés de formule I selon la revendication 1 et les sels physiologiquement inoffensifs de ceux-ci pour lutter contre l'ostéoporose, les tumeurs, la cachexie, l'athérosclérose, l'arthrite rhumatoïde, la sclérose en plaques, le diabète mellitus, la colite ulcérative, les maladies inflammatoires, les allergies, l'asthme, les maladies auto-immunes, le SIDA et les réactions de rejet au transplant.

7. Médicament de formule I selon la revendication 1 et les sels physiologiquement inoffensifs de celui-ci en tant qu'inhibiteur de phosphodiestérase IV.

8. Utilisation de composés de formule I selon la revendication 1 et/ou de sels physiologiquement inoffensifs de ceux-ci dans la fabrication d'un médicament.

9. Composés de formule I selon la revendication 1 et/ou sels physiologiquement inoffensifs de ceux-ci pour utilisation dans la lutte contre des maladies.
